(19) Europäisches Patentamt
European Patent Office
Office européen des brevets

(11) Numéro de publication: **0 141 713**
**B1**

(12) **FASCICULE DE BREVET EUROPEEN**

(45) Date de publication du fascicule du brevet: **30.05.90**

(21) Numéro de dépôt: **84402074.3**

(22) Date de dépôt: **16.10.84**

(51) Int. Cl.[5]: **C 07 D 277/46,** C 07 C 233/12, C 07 D 277/82, C 07 D 277/18, C 07 D 333/36, C 07 D 213/75, C 07 D 263/48, C 07 D 261/14, C 07 D 233/88, C 07 D 239/42, C 07 D 257/06 // C07D417/12, C07D215/56

(54) **Procédé de préparation des 3-quinoléine carboxamide 2- substitués.**

(30) Priorité: **20.10.83 FR 8316699**

(43) Date de publication de la demande: **15.05.85 Bulletin 85/20**

(45) Mention de la délivrance du brevet: **30.05.90 Bulletin 90/22**

(84) Etats contractants désignés: **AT BE CH DE FR GB IT LI NL**

(56) Documents cités: **EP-A-0 040 573**

(73) Titulaire: **ROUSSEL-UCLAF**
**35, boulevard des Invalides**
**F-75007 Paris (FR)**

(72) Inventeur: **Clemence, François**
**2, rue Turgot**
**F-75009 Paris (FR)**
Inventeur: **Le Martret, Odile**
**42, avenue de Versailles**
**F-75016 Paris (FR)**

(74) Mandataire: **Tonnellier, Marie-José et al**
**Département des Brevets ROUSSEL UCLAF B.P. no 9**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 141 713 B1

**Description**

La présente invention concerne un nouveau procédé de préparation des 3-quinoléine carboxamide 2-subtitués.

Ces 3-quinoléine carboxamides 2-substitués sont notamment les composés de formule (I') décrits dans le brevet européen n° 0 040 573, répondant à la formule suivante:

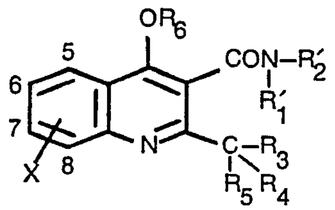

(I')

dans laquelle X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy, $R'_1$ représente un atome d'hydrogène ou un radical alcoyle renfermant de 1 à 4 atomes de carbone, $R'_2$ représente un atome d'hydrogène ou un radical choisi parmi les radicaux thiazolyle, 4,5-dihydrothiazolyle, pyridinyle, oxazolyle, isoxazolyle, imidazolyle, pyrimidyle, tétrazolyle, thiényle, benzothiazolyle et phényle, éventuellement substitués par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyle et g) le radical nitro, ou $R'_1$ et $R'_2$ forment ensemble l'un quelconque des cycles saturés ou insaturés mentionnés ci-dessus pour $R'_2$, ledit cycle étant alors relié à l'atome d'azote par une double liaison, $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que $R_3$, $R_4$ et $R_5$ ne représentent pas en même temps chacun un atome de fluor et $R_6$ représente un atome d'hydrogène, un radical alcoyle renfermant de 1 à 8 atomes de carbone ou un radical acyle renfermant de 2 à 8 atomes de carbone, ainsi que leurs sels d'addition avec les acides et les bases.

Le brevet européen cité précédemment décrit plusieurs procédés de préparation permettant d'obtenir les composés de formule (I').

L'un de ces procédés est caractérisé en ce que l'on soumet un composé de formule (VI):

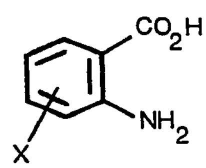

(VI)

dans laquelle X conserve la signification donnée précédemment, à l'action d'un acide de formule (VII):

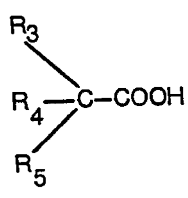

(VII)

dans laquelle $R_3$, $R_4$, $R_5$ conservent les significations données précédemment ou d'un dérivé fonctionnel de l'acide de formule (VII), pour obtenir un composé de formule (VIII):

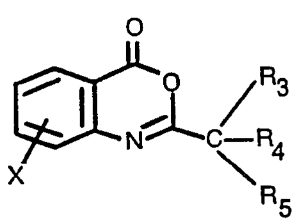

(VIII)

dans laquelle X conserve la signification précédente, que l'on soumet à l'action d'un composé de formule (IX):

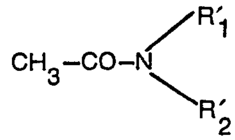

(IX)

dans laquelle R'$_1$ et R'$_2$ conservent les significations données précédemment, pour obtenir le composé de formule (X):

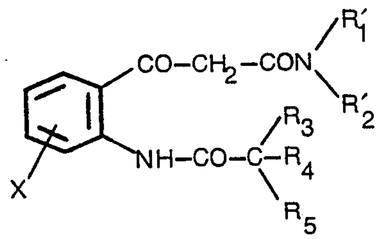

(X)

dans laquelle R'$_1$, R'$_2$, $R_3$, $R_4$ et $R_5$ conservent leur signification précédente, que l'on cyclise en présence d'un agent alcalin, pour obtenir le composé de formule (I'$_A$):

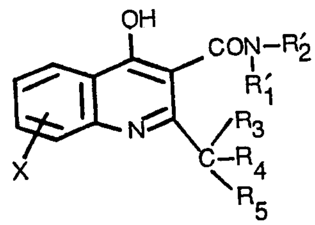

(I'$_A$)

dans laquelle X, R'$_1$, R'$_2$, $R_3$, $R_4$ et $R_5$ sont définis comme précédemment, correspondant à un produit de formule (I') dans laquelle $R_6$ représente un atome d'hydrogène, produit de formule (I'$_A$) que l'on soumet, si désiré, à l'action d'un agent d'éthérification ou d'estérification, pour obtenir un produit de formule (I'$_B$):

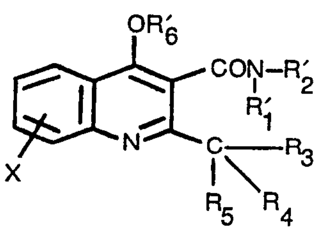

(I'$_B$)

dans laquelle X, R'$_1$, R'$_2$, $R_3$, $R_4$ et $R_5$ sont définis comme précédemment et R'$_6$ a les valeurs indiquées précédemment pour $R_6$, à l'exception d'hydrogène, produit de formule (I'$_A$) ou (I'$_B$) que, si désiré, l'on soumet à l'action d'un acide ou d'une base pour en former le sel.

Cet "ancien procédé", ainsi qu'il est indiqué plus loin, ne donne pas toute satisfaction. Il a donc été recherché une nouvelle voie d'accès aux produits de formule (I') ci-dessus. Cette nouvelle voie conduit intermédiairement à des composés nouveaux répondant à la formule (C):

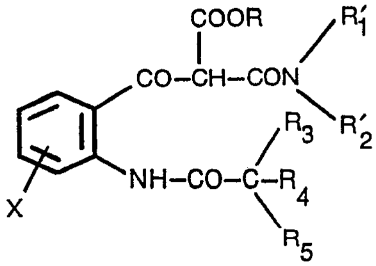

(C)

dans laquelle X, R'$_1$, R'$_2$, $R_3$, $R_4$ et $R_5$ conservent leurs significations précédentes et R représente un radical alcoyle renfermant de 1 à 8 atomes de carbone.

Dans les produits de formule (C), R est de préférence un radical méthyle, éthyle, n-propyle ou isopropyle, R'$_1$ est de préférence un atome d'hydrogène et R'$_2$ est de préférence un radical thiazolyle.

L'invention a en outre pour objet un procédé de préparation des composés de formule (C) telle que définie précédemment, caractérisé en ce que l'on soumet en présence d'une base, un composé de formule (A):

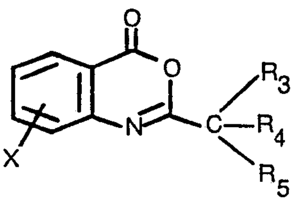

(A)

dans laquelle X, $R_3$, $R_4$ et $R_5$ conservent leurs significations précédentes, à l'action d'un composé de formule (B):

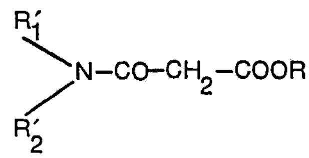

(B)

dans laquelle $R'_1$, $R'_2$ et R ont les significations déjà données, pour obtenir un composé de formule (C).

La base utilisée en cours du procédé de l'invention est destinée à préparer, au départ du composé (B), le monoanion de formule:

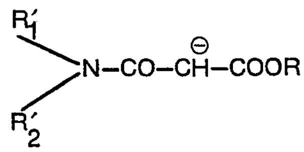

lequel réagit ensuite sur le composé de formule (A). L'utilisation d'une telle base ne conduit qu'à la formation d'un monoanion même lorsque dans le composé de formule (B), $R'_1$ est un atome d'hydrogène.

Parmi les bases pouvant être utilisées pour obtenir le monoanion du composé de formule (B), on citera, de préférence, un hydroxyde de métal alcalin et, tout particulièrement l'hydroxyde de sodium. On peut encore citer l'hydrure de sodium ou de potassium, les alcoolates des métaux alcalins, par exemple le méthylate de sodium, le butylate de potassium ou l'éthylate de magnésium ou des amidures comme l'amidure de potassium ou de sodium.

La réaction est effectuée de préférence au sein du tétrahydrofuranne. On peut cependant également utiliser l'acétate d'éthyle, le dioxanne, le toluène, le diméthylformamide ou des mélanges de ces solvants.

L'invention a tout particulièrement pour objet un procédé tel que défini précédemment, caractérisé en ce que la base utilisée est l'hydroxyde de sodium, que l'on opère au sein du tétrahydrofuranne et à température ambiante.

Il peut être intéressant afin d'accélérer le processus de la réaction, d'utiliser deux équivalents de base.

L'invention a également pour objet une application des composés de formule (C) telle que définie précédemment, caractérisée en ce que l'on soumet un composé de formule (C) à une décarbalcoxylation en milieu acide pour obtenir un composé de formule (D):

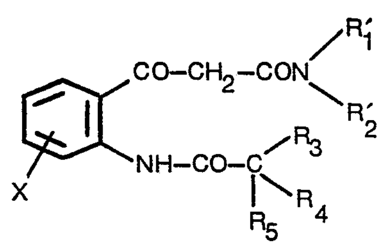

(D)

dans laquelle X, $R'_1$, $R'_2$, $R_3$, $R_4$ et $R_5$ ont la signification précitée.

Dans des conditions préférentielles d'exécution de l'application, objet de l'invention, on opère la décarbalcoxylation par chauffage, en présence d'un mélange d'acide sulfurique et d'acide dichloracétique. On peut encore opérer en présence d'acide trifluoroacétique, d'un mélange acide formique-acide méthane sulfonique, ou encore d'un mélange acide acétique-acide sulfurique, chauffé à une température de l'ordre de 100°C.

Les composés de formule (D) sont des intermédiaires dans la préparation des 3-quinoléine carboxamides 2-substitués de formule (I') définie précédemment.

Une cyclisation des composés de formule (D), effectuée en présence d'un agent alcalin, suivie, le cas

échéant, d'une estérification ou d'une éthérification, conduit en effet aux composés de formule (I') que l'on peut, en outre, salifier par action d'une base. De telles réactions sont décrites dans le brevet européen n° 0 040 573.

En ce qui concerne la désignation des composés utilisés ou préparés respectivement dans le brevet européen n° 0 040 573 et la présente demande, les différences sont les suivantes:

Les composés de formule (A), composés de départ utilisés dans la présente demande, sont les composés de formule (VIII) du brevet n° 0 040 573. Les valeurs préférentielles pour les substituants $R_3$, $R_4$, $R_5$ et X sont les mêmes que celles indiquées dans le brevet européen n° 0 040 573.

Les composés de formule (A) réagissent avec les composés de formule (B), différents des composés de formule (IX), utilisés dans le brevet européen n° 0 040 573.

Les produits de formule (D), obtenus après décarboalcoxylation des produits de formule (C) sont les produits de formule (X) du procédé du du brevet européen n° 0 040 573.

Le nouveau procédé de préparation des composés de formule (C), selon l'invention, ainsi que leur application à la préparation des composés de formule (D), présentent par rapport au procédé connu antérieurement par le brevet européen n° 0 040 573, un certain nombre d'avantages qui se sont avérés déterminants.

Ces avantages sont notamment les suivants:

Dans l'ancien procédé, la réaction entre le composé de formule (VIII) et le composé de formule (IX) a lieu en présence d'une base forte qui permet la formation de l'anion du composé (IX) lequel réagit ensuite sur le composé de formule (VIII).

Une telle base forte est notamment un organolithien comme le n-butyllithium ou un amidure de lithium tel que le diisopropyl amidure de lithium. Or, ce type de réactif présente l'inconvénient d'être coûteux et par ailleurs, demande à être manipulé avec précaution. En effet, l'anion formé étant très réactif, sa réaction sur le composé de formule (VIII) doit être modérée en opérant à une basse température de l'ordre de −70°C. Ce type de conditions opératoires est peu propice à la transposition d'un procédé au plan industriel.

En outre, dans le cas des composés de formule (IX), pour lesquels $R'_1$ est un atome d'hydrogène, il se forme un dianion de formule:

$$CH_2^{\ominus}-\underset{\underset{O}{\|}}{C}-N^{\ominus}-R'_2$$

La formation de ce dianion nécessite donc une grande quantité de base puisqu'elle requiert deux équivalents de réactif coûteux. Il était donc intéressant de trouver une nouvelle synthèse permettant de remplacer la base telle que le n-butyllithium par un réactif moins onéreux, de sécurité d'emploi plus grande, que l'on puisse employer en moins grande quantité et permettant de supprimer le refroidissement à très basse température.

Dans le procédé de la présente invention, la base utilisée lors de la réaction entre le composé de formule (A) et le composé de formule (B) et permettant d'obtenir le monoanion de composé de formule (B), est une base moins forte que celle employée dans l'ancien procédé et surtout d'un usage infiniment plus commode et moins coûteux.

De plus, le monoanion formé est beaucoup moins réactif que l'anion formé au cours de l'ancien procédé.

Ceci a pour conséquence que la réaction entre le composé de formule (A) et le composé de formule (B) ne nécessite pas de refroidissement à très basse température ou de chauffage du milieu réactionnel.

La suppression du refroidissement notamment, constitue en lui-même une amélioration importante, (contrôle plus facile, réduction du coût) dans la mise en oeuvre de la synthèse.

Comme indiqué plus haut, la base préférée est l'hydroxyde de sodium, utilisé à température ambiante, dans le tétrahydrofuranne.

Par rapport aux agents basiques forts utilisés dans le procédé du brevet européen n° 0 040 573, l'utilisation des agents basiques selon le procédé de la présente invention et notamment de la soude, présente en outre l'avantage de conduire au composé souhaité sans formation, par attaque parasite de l'anion intermédiaire sur le carbone en position 2 de la benzoxazine, du dérivé de formule:

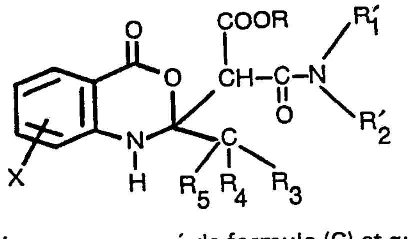

Ce dérivé qui ne peut s'isomériser en composé de formule (C) et qui constitue par conséquent un sous produit de la réaction tout a fait inutile et qu'il est nécessaire de séparer du milieu réactionnel, est formé dans des proportions notables si l'on utilise les agents basiques forts du brevet européen n° 0 040 573.

La décarboalcoxylation du composé de formule (C) en composé de formule (D), réalisée dans l'application selon l'invention, n'était pas nécessaire dans l'ancien procédé.

En dépit de ce stade supplémentaire, il est surprenant de constater que le rendement en produit (D) est supérieur à celui obtenu avec l'ancien procédé.

Comme indiqué précédemment, les composés de formule (D) sont des intermédiaires dans la synthèse des 3-quinoléine carboxamides 2-substitués décrits dans le brevet européen n° 0 040 573.

Les composés de formule (D) peuvent également être utilisés comme intermédiaires dans la synthèse d'autres 3-quinoléine carboxamides 2-substitués, décrits dans le brevet belge 896 941 et comportant sur la chaîne en position 2, une fonction hydroxy éventuellement estérifiée.

Les exemples suivants illustrent l'invention sans toutefois la limiter.

Exemple 1

2-(dichloroacétylamino) β-oxo N-(2-thiazolyl) 3-trifluorométhyl benzène propanamide

*Stade A*: 2-[(2,2-dichloro 1-oxo éthyl)amino] β-oxo α-[(2-thiazolyl amino)carbonyl] 3-(trifluorométhyl)benzène propanoate d'éthyle

On mélange 75 $cm^3$ de tétrahydrofuranne et 2,74 g d'hydrure de sodium à 55% en dispersion dans l'huile, puis ajoute, entre +14 et +15°C, goutte à goutte 12,85 g de 3-(2-thiazolylamino) 3-oxo propanoate d'éthyle (décrit Journal Am. Chem. Soc. *64*.2712.3, 1942) et 250 $cm^3$ de tétrahydrofuranne. On refroidit à +5°C et ajoute à cette température 17,88 g de 2-(dichlorométhyl) 8-(trifluorométhyl) 4H-3,1-benzoxazine 4-one préparé à l'exemple 12 (stade A) du brevet européen n° 0 040 573, et 250 $cm^3$ de tétrahydrofuranne, agite pendant 15 minutes, verse la solution sur un litre d'eau additionnée de 100 $cm^3$ d'HCl 2N, extrait à l'éther, sèche les extraits organiques obtenus et concentre sous pression réduite. On fait cristalliser le résidu obtenu dans l'éther puis essore, lave à l'éther, sèche sous pression réduite et obtient 22 g de produit attendu fondant à 180—182°C.

*Stade B*: Décarbéthoxylation

Obtention du 2-(dichloroacétylamino) β-oxo N-(2-thiazolyl) 3-trifluorométhyl benzène propanamide.

On mélange 5,1 g de produit obtenu au stade A, 25 $cm^3$ d'acide acétique pur et 0,53 $cm^3$ d'acide sulfurique concentré, chauffe à 100°C pendant 45 minutes jusqu'à arrêt du dégagement gazeux. On ajoute 80 $cm^3$ d'eau à la solution obtenue, essore, lave à l'eau, sèche sous pression réduite à 100°C et obtient 4 g de produit attendu fondant vers 216—218°C.

Exemple 2

2-(dichloroacétylamino) β-oxo N-(2-thiazolyl) 3-trifluorométhyl benzène propanamide

*Stade A*: 2-[(2,2-dichloro 1-oxo éthyl)amino] β-oxo α-[(2-thiazolyl amino)carbonyl] 3-(trifluorométhyl)benzène propanoate d'éthyle

On mélange à 20°C, 150 g de 2-dichlorométhyl 8-trifluorométhyl 4H-3,1-benzoxazine 4-one, 120 g de 3-(2-thiazolylamino) 3-oxo propanoate d'éthyle, 1,5 l de tétrahydrofuranne, agite pendant 30 minutes puis ajoute, en maintenant à 20°C, 40 g de soude en écailles. On maintient sous agitation à 20°C, 40 g de soude en écailles. On maintient sous agitation à 20°C pendant 2 heures, puis verse dans un mélange de 4,5 d'eau et 150 $cm^3$ d'acide chlorhydrique concentré, agite à 0, +5°C pendant 30 minutes puis essore les cristaux, les rince à l'eau et les sèche. On obtient 255 g de produit attendu. F = 90°C.

*Stade B*: 2-(dichloroacétylamino) β-oxo N-(2-thiazolyl) 3-trifluorométhyl benzène propanamide

On mélange 100 g de produit obtenu au stade A avec 180 $cm^3$ d'acide dichloroacétique, on agite à 20—25°C jusqu'à dissolution puis ajoute lentement à 20—25°C, 5 $cm^3$ d'acide sulfurique concentré. On obtient une solution A. On mélange à 25°C, 100 $cm^3$ d'acide dichloroacétique et 5 $cm^3$ d'acide sulfurique concentré, porte à 107°C et ajoute lentement, sous gaz inerte, la solution A ci-dessus en agitant à 105—7°C pendant 30 minutes, refroidit à 20°C et verse lentement dans 2 litres d'eau. On agite la suspension obtenue à 20°C pendant 18 heures, essore les cristaux, les lave à l'eau et les sèche. On obtient le produit attendu identique à celui obtenu à l'exemple 1.

Exemple 3

2-[(2-chloro 1-oxo 3-méthylbutyl)amino] β-oxo N-(2-thiazolyl) 3-trifluorométhyl benzène propanamide

*Stade A*: 2-[(2-chloro 1-oxo 3-méthylbutyl)amino] β-oxo α-[(2-thiazolylamino)carbonyl] 3-trifluorométhyl benzène propanoate d'éthyle

On mélange sous gaz inerte 5,52 g d'hydrure de sodium en dispersion à 55% dans l'huile et 173 $cm^3$ de tétrahydrofuranne. On ajoute ensuite lentement à 20°C, une solution de 26,22 g de 3-(2-thiazolylamino) 3-oxo propanoate d'éthyle dans 690 $cm^3$ de tétrahydrofuranne, agite pendant 10 minutes puis ajoute lentement, à 20°C, une solution de 34,5 g de 2-(1-chloro 2-méthyl propyl) 8-trifluorométhyl 4H-3,1-benzoxazine 4-one (préparée selon le procédé décrit dans le brevet européen n° 0 040 573) dans 210 $cm^3$ de tétrahydrofuranne. On agite à 20°C pendant 35 minutes, verse dans 200 $cm^3$ d'acide chlorhydrique 2N, extrait à l'éther, lave la phase éthérée à l'eau, la sèche et évapore le solvant. On empâte le résidu dans l'éther, sèche et obtient 42,35 g de produit attendu. F = 160°C. On obtient ensuite un 2ème jet de 8,2 g de produit attendu. F = 160°C.

*Stade B*: 2-[(2-chloro 1-oxo 3-méthylbutyl)amino] β-oxo N-(2-thiazolyl) 3-trifluorométhyl benzène propanamide

On mélange 50,4 g de produit obtenu au stade A, 252 $cm^3$ d'acide acétique et 5,04 $cm^3$ d'acide sulfurique concentré. On porte la solution obtenue à 100°C pendant 50 minutes, ajoute 2 litres d'eau puis 500 $cm^3$ d'acétone, agite à 20°C pendant 2 heures 30 minutes, verse à nouveau 1 litre d'eau, agite pendant 15 minutes, essore et sèche les cristaux formés. On obtient 35 g de produit attendu. F = 186°C.

Exemple 4

2-[(2-chloro 1-oxo butyl)amino] β-oxo N-(2-thiazolyl) 8-trifluorométhyl benzène propanamide

*Stade A*: 2-[(2-chloro 1-oxo butyl) amino] β-oxo α-[(2-thiazolylamino)carbonyl] 3-trifluorométhyl benzène propanoate d'éthyle

On mélange sous gaz inerte 3,83 g d'hydrure de sodium en dispersion à 55% dans l'huile, dans 100 $cm^3$ de tétrahydrofuranne. On ajoute lentement sous agitation, à 20°C une solution de 17,92 g de 3-(2-thiazolylamino) 3-oxo propanoate d'éthyle dans 400 $cm^3$ de tétrahydrofuranne. On agite la solution obtenue à 20°C pendant 15 minutes puis ajoute une solution de 24,4 g de 2-(1-chloropropyl) 8-trifluorométhyl 4H-3,1-benzoxazine 4-one (préparée selon le procédé décrit dans le brevet belge n° 896 941) dans 250 $cm^3$ de tétrahydrofuranne. On verse la solution obtenue sur un mélange de 1 litre d'eau et 100 $cm^3$ d'acide chlorhydrique 2N, extrait à l'éther, lave la phase éthérée à l'eau, la sèche et évapore le solvant. On empâte le résidu dans l'éther, sèche les cristaux formés et obtient 34,5 g de produit attendu. F = 170°C.

*Stade B*: 2-[(2-chloro 1-oxo butyl)amino] β-oxo N-(2-thiazolyl) 8-trifluorométhyl benzène propanamide

On mélange le produit obtenu au stade A avec 175 $cm^3$ d'acide acétique et ajoute 3,5 $cm^3$ d'acide sulfurique concentré. On porte à 100°C pendant 45 minutes, refroidit, verse sur un mélange de 400 $cm^3$ d'eau et 40 $cm^3$ d'acétone, agite pendant 1 heure, essore les cristaux formés, les lave à l'eau et les sèche. On obtient 22,9 g de produit attendu. F = 190°C.

Exemple 5

2-[(2-chloro 1-oxo 3,3-diméthylbutyl)amino] β-oxo N-(2-thiazolyl) 3-trifluorométhyl benzène propanamide

*Stade A*: 2-[(2-chloro 1-oxo 3,3-diméthylbutyl)amino] β-oxo α-[(2-thiazolylamino)carbonyl] 3-trifluorométhyl benzène propanoate d'éthyle

On opère de manière analogue à celle décrite au stade A de l'exemple 4, en utilisant au départ 7,7 g de 3-(2-thiazolylamino) 3-oxo propanoate d'éthyle et 10,55 g de 2-(1-chloro 2,2-diméthyl propyl) 8-trifluorométhyl 4H-3,1-benzoxazine 4-one (préparée selon le procédé décrit dans le brevet européen n° 0 040 573). On obtient 15,6 g de produit attendu. F = 180°C.

*Stade B*: 2-[(2-chloro 1-oxo 3,3-diméthylbutyl)amino] β-oxo N-(2-thiazolyl) 3-trifluorométhyl benzène propanamide

On opère de manière analogue à celle décrite au stade B de l'exemple 4, en utilisant au départ 14 g du produit obtenu au stade A et un mélange de 70 $cm^3$ d'acide acétique et 1,4 $cm^3$ d'acide sulfurique concentré. On obtient 9,9 g de produit attendu. F = 206°C.

**Revendications**

1. Procédé de préparation des 3-quinoléine carboxamide 2-substitués de formule générale (I'):

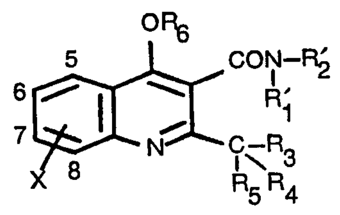

(I')

dans laquelle X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy, $R'_1$ représente un atome d'hydrogène ou un radical alcoyle renfermant de 1 à 4 atomes de carbone, $R'_2$ représente un atome d'hydrogène ou un radical choisi parmi les radicaux thiazolyle, 4,5-dihydrothiazolyle, pyridinyle, oxazolyle, isoxazolyle, imidazolyle, pyrimidyle, tétrazolyle, thiényle, benzothiazolyle et phényle, éventuellement substitués par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyl et g) le radical nitro, ou $R'_1$ et $R'_2$

forment ensemble l'un quelconque des cycles saturés ou insaturés mentionnés ci-dessus pour $R'_2$, ledit cycle étant alors relié à l'atome d'azote par une double liaison, $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que $R_3$, $R_4$ et $R_5$ ne représentent pas en même temps chacun un atome de fluor et $R_6$ représente un atome d'hydrogène, un radical alcoyle renfermant de 1 à 8 atomes de carbone ou un radical acyle renfermant de 2 à 8 atomes de carbone, ainsi que leurs sels d'addition avec les acides et les bases, comprenant le fait que l'on soumet à une décarboalcoxylation, en milieu acide, un composé de formule (C):

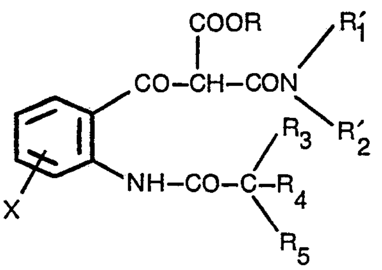

(C)

dans laquelle X, $R'_1$, $R'_2$, $R_3$, $R_4$ et $R_5$ ont la signification indiquée ci-dessus et R représente un radical alcoyle renfermant de 1 à 8 atomes de carbone pour obtenir un composé de formule (D):

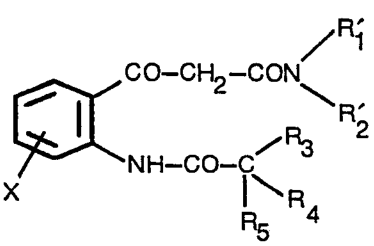

(D)

dans laquelle X, $R'_1$, $R'_2$, $R_3$, $R_4$ et $R_5$ sont définis comme précédemment, produit que l'on cyclise en présence d'un agent alcalin, pour obtenir le composé de formule ($I'_A$):

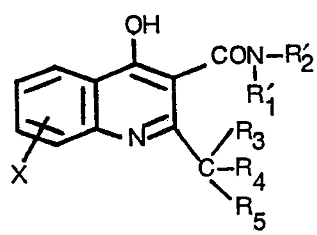

($I'_A$)

dans laquelle X, $R'_1$, $R'_2$, $R_3$, $R_4$ et $R_5$ sont définis comme précédemment, correspondant à un produit de formule (I') dans laquelle $R_6$ représente un atome d'hydrogène, produit de formule ($I'_A$) que l'on soumet, si désiré, à l'action d'un agent d'éthérification ou d'estérification, pour obtenir un produit de formule ($I'_B$):

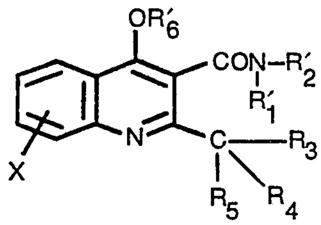

($I'_B$)

dans laquelle X, $R'_1$, $R'_2$, $R_3$, $R_4$ et $R_5$ sont définis comme précédemment et $R'_6$ a les valeurs indiquées précédemment pour $R_6$, à l'exception d'hydrogène, produit de formule ($I'_A$) ou ($I'_B$) que, si désiré, l'on soumet à l'action d'un acide ou d'une base pour en former le sel, procédé caractérisé en ce que les composés de formule (C) sont préparés en soumettant en présence d'une base, un composé de formule (A):

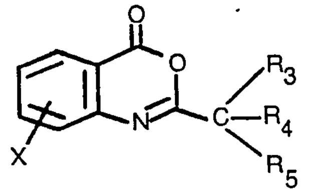

(A)

dans laquelle X, $R_3$, $R_4$ et $R_5$ conservent leurs significations précédentes, à l'action d'un composé de formule (B):

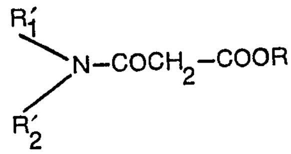

(B)

dans laquelle R, $R'_1$ et $R'_2$ ont les significations déjà données.

2. Procédé selon la revendication 1, caractérisé en ce que la base utilisée est l'hydroxyde de sodium.

3. Procédé selon la revendication 2, caractérisé en ce que l'on opère au sein du tétrahydrofuranne à température ambiante.

4. Procédé selon l'une quelconque des revendications 1 à 3, caractérisé en ce que l'on utilise au départ un composé de formule (B) dans laquelle R représente un radical méthyle, éthyle, n-propyle ou isopropyle.

5. Procédé selon l'une quelconque des revendications 1 à 4, caractérisé en ce que l'on utilise au départ un composé de formule (B) dans laquelle $R'_1$ représente un atome d'hydrogène et $R'_2$ représente un radical thiazolyle.

**Patentansprüche**

1. Verfahren zur Herstellung von 2-subst.-3-Chinolincarboxamiden der allgemeinen Formel (I')

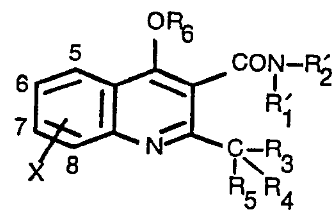

(I')

worin X in 5-, 6-, 7- oder 8-Stellung für ein Wasserstoffatom, ein Halogenatom, einen linearen oder verzweigten Alkylrest mit 1 bis 5 Kohlenstoffatomen, eine lineare oder verzweigte Alkoxygruppe mit 1 bis 4 Kohlenstoffatomen, einen Trifluormethylrest, einen Trifluormethylthiorest oder einen Trifluormethoxyrest steht, $R'_1$ ein Wasserstoffatom oder einen Alkylrest mit 1 bis 4 Kohlenstoffatomen wiedergibt, $R'_2$ ein Wasserstoffatom oder einen Rest, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Thienyl-, Benzothiazolyl- und Phenylresten, bedeutet, die gegebenenfalls durch einen oder mehrere Reste substituiert sind, ausgewählt unter der Gruppe, bestehend aus (a) den Halogenen, (b) den Alkylresten mit 1 bis 4 Kohlenstoffatomen, die unsubstituiert sind oder substituiert sind durch eine Amino-, Alkylamino- oder Dialkylaminogruppe, deren Alkylreste 1 bis 3 Kohlenstoffatome enthalten, (c) dem Phenylrest, (d) den Alkoxyresten mit 1 bis 4 Kohlenstoffatomen, (e) der Hydroxygruppe, (f) dem Trifluormethylrest und (g) der Nitrogruppe, oder $R'_1$ und $R'_2$ gemeinsam einen der vorstehend für $R'_2$ genannten, gesättigten oder ungesättigten Ringe bilden, wobei dieser Ring dann an das Stickstoffatom über eine Doppelbindung gebunden ist, $R_3$ ein Wasserstoffatom, ein Halogenatom oder eine Alkylgruppe mit 1 bis 4 Kohlenstoffatomen bedeutet, $R_4$ für ein Wasserstoffatom oder ein Halogenatom steht, $R_5$ ein Halogenatom bedeutet, unter der Bedingung, daß $R_3$, $R_4$ und $R_5$ nicht gleichzeitig jeweils ein Fluoratom darstellen, und $R_6$ ein Wasserstoffatom, eine Alkylgruppe mit 1 bis 8 Kohlenstoffatomen oder eine Acylgruppe mit 2 bis 8 Kohlenstoffatomen bedeutet, sowie von deren Additionssalzen mit Säuren und Basen, bei dem man in saurem Milieu eine Verbindung der Formel (C)

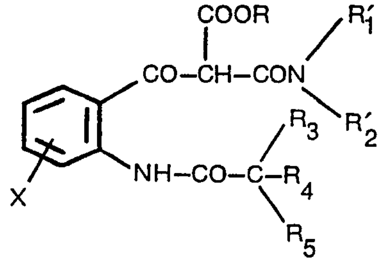

(C)

worin X, $R'_1$, $R'_2$, $R_3$, $R_4$ und $R_5$ die vorstehend angegebene Bedeutung besitzen und R einen Alkylrest mit 1 bis 8 Kohlenstoffatomen wiedergibt, einer Decarbalkoxylierung unterzieht, um zu einer Verbindung der Formel (D)

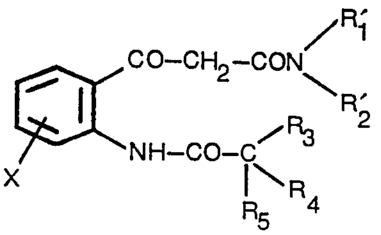

(D)

zu gelangen, worin X, $R'_1$, $R'_2$, $R_3$, $R_4$ und $R_5$ wie vorstehend definiert sind, welche man in Gegenwart eines alkalischen Mittels cyclisiert, um die Verbindung der Formel ($I'_A$)

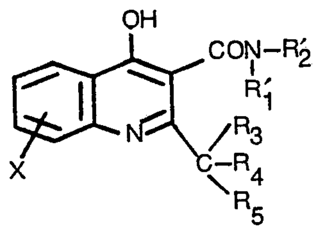

($I'_A$)

zu erhalten, worin X, $R'_1$, $R'_2$, $R_3$, $R_4$ und $R_5$ wie vorstehend definiert sind, entsprechend einem Produkt der Formel (I'), in dem $R_6$ für ein Wasserstoffatom steht, das Produkt der Formel ($I'_A$) gewünschtenfalls der Einwirkung eines Veretherungs- oder Veresterungsmittels unterzieht, um zu einem Produkt der Formel ($I'_B$)

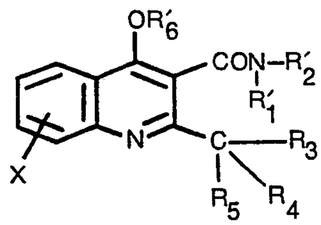

($I'_B$)

zu gelangen, worin X, $R'_1$, $R'_2$, $R_3$, $R_4$ und $R_5$ wie vorstehend definiert sind und $R'_6$ die vorstehend für $R_6$ angegebenen Bedeutungen mit Ausnahme von Wasserstoff besitzt, und das Produkt der Formel ($I'_A$) oder ($I'_B$) gewünschtenfalls der Einwirkung einer Säure oder einer Base unterzieht, um hieraus das Salz zu bilden, dadurch gekennzeichnet, daß die Verbindungen der Formel (C) hergestellt werden, indem man in Gegenwart einer Base eine Verbindung der Formel (A)

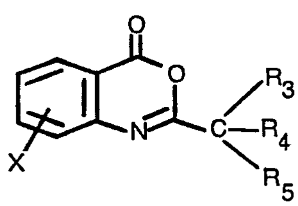

(A)

worin X, $R_3$, $R_4$ und $R_5$ die vorstehenden Bedeutungen besitzen, der Einwirkung einer Verbindung der Formel (B)

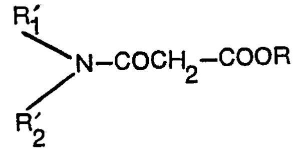

(B)

worin R, $R'_1$ und $R'_2$ die vorstehend angegebenen Bedeutungen besitzen, unterzieht.

2. Verfahren gemäß Anspruch 1, dadurch gekennzeichnet, daß die verwendete Base Natriumhydroxid ist.

3. Verfahren gemäß Anspruch 2, dadurch gekennzeichnet, daß man in Tetrahydrofuran bei Raumtemperatur arbeitet.

4. Verfahren gemäß einem der Ansprüche 1 bis 3, dadurch gekennzeichnet, daß man von einer Verbindung der Formel (B) ausgeht, in der R für einen Methyl-, Ethyl-, n-Propyl- oder Isopropylrest steht.

5. Verfahren gemäß einem der Ansprüche 1 bis 4, dadurch gekennzeichnet, daß man von einer Verbindung der Formel (B) ausgeht, worin $R'_1$ für ein Wasserstoffatom steht und $R'_2$ für einen Thiazolylrest steht.

**Claims**

1. Process for the preparation of substituted 3-quinoleine carboxamide-2 of the general formula (I'):

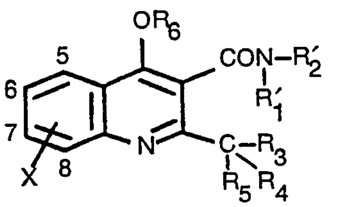

(I')

in which X at position 5, 6, 7 or 8 represents a hydrogen atom, a halogen atom, a linear or branched alkyl radical containing from 1 to 5 carbon atoms, a linear or branched alkoxy radical containing from 1 to 4 carbon atoms, a trifluoromethyl radical, a trifluoromethylthio radical or a trifluoromethoxy radical, $R'_1$ represents a hydrogen atom or an alkyl radical containing from 1 to 4 carbon atoms, $R'_2$ represents a hydrogen atom or a radical selected from the radicals thiazolyl, 4,5-dihydrothiazolyl, pyridinyl, oxazolyl, isooxazolyl, imidazolyl, pyrimidyl, tetrazolyl, thienyl, benzothiazolyl and phenyl, optionally substituted by one or more radicals selected from the group constituted by a) halogens, b) alkyl radicals containing from 1 to 4 carbon atoms, not substituted, or substituted by an amino, alkylamino or dialkylamino radical of which the alkyl radicals contain from 1 to 3 carbon atoms, c) a phenyl radical, d) alkoxy radicals containing from 1 to 4 carbon atoms, e) a hydroxy radical, f) a trifluoromethyl radical and g) a nitro radical, or $R'_1$ and $R'_2$ together form any one of the saturated or unsaturated rings mentioned above for $R'_2$, said ring then being joined to the nitrogen atom by a double bond, $R_3$ represents a hydrogen atom, a halogen atom, an alkyl radical containing from 1 to 4 carbon atoms, $R_4$ represents a hydrogen atom or a halogen atom, $R_5$ represents a halogen atom on condition that $R_3$, $R_4$ and $R_5$ do not each simultaneously represent a fluorine atom and $R_6$ represents a hydrogen atom, an alkyl radical containing from 1 to 8 carbon atoms or an acyl radical containing from 2 to 8 carbon atoms, as well as their addition salts with acids and bases, including the fact that a compound of formula (C):

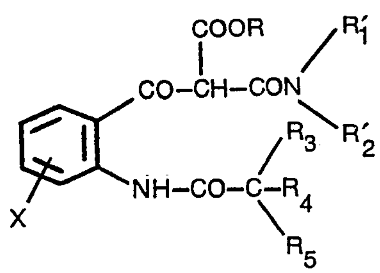

(C)

in which X, $R'_1$, $R'_2$, $R_3$, $R_4$ and $R_5$ have the meaning indicated above and R represents an alkyl radical containing from 1 to 8 carbon atoms, is submitted to a decarboalkoxylation in an acid medium to obtain a compound of formula (D):

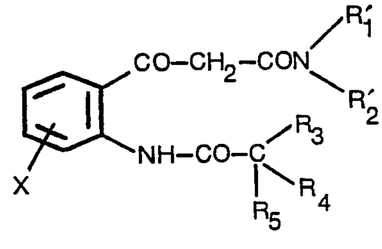

(D)

in which X, $R'_1$, $R'_2$, $R_3$, $R_4$ and $R_5$ are defined as above, which product is cyclised in the presence of an alkaline agent to obtain the compound of formula ($I'_A$):

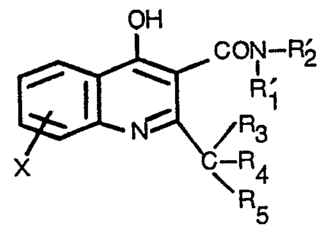

($I'_A$)

in which X, $R'_1$, $R'_2$, $R_3$, $R_4$ and $R_5$ are defined as above, corresponding to a product of formula (I') in which $R_6$ represents a hydrogen atom, which product of formula ($I'_A$) is submitted, if desired, to the action of an etherification or esterification agent to obtain a product of formula ($I'_B$):

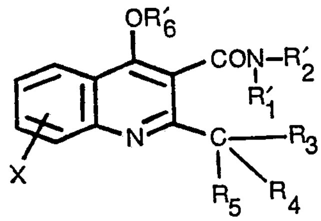

($I'_B$)

in which X, $R'_1$, $R'_2$, $R_3$, $R_4$ and $R_5$ are defined as above and $R'_6$ has the meanings previously indicated for $R_6$, except for hydrogen, which product of formula ($I'_A$) or ($I'_B$), if desired, is submitted to the action of an acid or a base to form the salt, which process is characterized in that the compounds of formula (C) are prepared in the presence of a base by submitting a compound of formula (A):

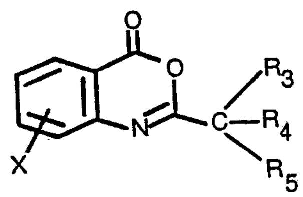

(A)

in which X, $R_3$, $R_4$ and $R_5$ keep their previous meanings, to the action of a compound of formula (B):

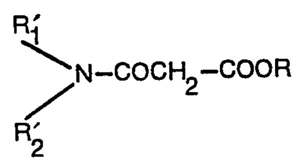

(B)

in which R, $R'_1$ and $R'_2$ have the meanings already given.

2. Process according to claim 1, characterized in that the base used is sodium hydroxide.

3. Process according to claim 2, characterized in that the procedure is carried out in tetrahydrofuran at ambient temperature.

4. Process according to one of claims 1 to 3 characterized in that at the start a compound of formula (B) is used, in which R represents a methyl, ethyl, n-propyl or isopropyl radical.

5. Process according to one of claims 1 to 4, characterized in that at the start a compound of formula (B) is used, in which $R'_1$ represents a hydrogen atom and $R'_2$ represents a thiazolyl radical.